(19) Europäisches Patentamt
European Patent Office
Office européen des brevets

(11) **EP 1 666 448 B1**

(12) **EUROPÄISCHE PATENTSCHRIFT**

(45) Veröffentlichungstag und Bekanntmachung des Hinweises auf die Patenterteilung:
**20.08.2014 Patentblatt 2014/34**

(21) Anmeldenummer: **04772893.6**

(22) Anmeldetag: **31.08.2004**

(51) Int Cl.:
*A61K 8/39* (2006.01)     *A61K 8/86* (2006.01)
*A61Q 1/02* (2006.01)     *A61Q 1/06* (2006.01)
*A61Q 5/02* (2006.01)     *A61Q 5/06* (2006.01)
*A61Q 19/00* (2006.01)    *A61Q 19/10* (2006.01)
*C07C 69/33* (2006.01)

(86) Internationale Anmeldenummer:
**PCT/JP2004/013079**

(87) Internationale Veröffentlichungsnummer:
**WO 2005/023751 (17.03.2005 Gazette 2005/11)**

(54) **VERESTERUNGSPRODUKT UND KOSMETIKUM**

ESTERIFICATION PRODUCT AND COSMETIC

PRODUIT D'ESTERIFICATION ET COSMETIQUE

(84) Benannte Vertragsstaaten:
**DE FR GB**

(30) Priorität: **05.09.2003 JP 2003313715**

(43) Veröffentlichungstag der Anmeldung:
**07.06.2006 Patentblatt 2006/23**

(73) Patentinhaber: **Sakamoto Yakuhin Kogyo Co., Ltd.**
**Osaka-shi**
**Osaka 541-0047 (JP)**

(72) Erfinder:
• **OKI, J.,**
**R&D Lab. Sakamoto Yakuhin Kogyo Co., Ltd.**
**Osaka 595-0075 (JP)**
• **NOGUSHI, Y.,**
**R&DLab. Sakamoto Yakuhin Kogyo Co.Ltd**
**Osaka 595-0075 (JP)**

(74) Vertreter: **Jeck, Anton et al**
**Jeck Fleck Herrmann**
**Patentanwälte**
**Postfach 14 69**
**71657 Vaihingen/Enz (DE)**

(56) Entgegenhaltungen:
**WO-A1-2004/002438     JP-A- 7 308 560**
**JP-A- 8 143 513     JP-A- 8 217 723**
**JP-A- 8 217 725**

• **DATABASE WPI Week 199536 Derwent Publications Ltd., London, GB; AN 1995-273029 XP002404169 & JP 07 173380 A (MITSUBISHI CHEM CORP) 11. Juli 1995 (1995-07-11)**

**Beschreibung**

Die vorliegende Erfindung betrifft Veresterungsprodukte und insbesondere Fettsäureester des Polyglyzerols, die durch Veresterung von Polygrizerol und Fettsäure gebildet werden, und einen diesen Ester enthaltenden Kosmetikartikel.

Ölrohmaterialien werden als Hauptrohmaterialien von sehr verschiedenen Kosmetikartikeln und als wichtige Bestandteile von Cremes und Lotions verwendet. Die für die Kosmetikartikel verwendeten Ölrohmaterialien werden für solche Zwecke verwendet, wie die Verhinderung einer Schweißabsonderung der Haut und die Verbesserung des Gebrauchsgefühls. Die Ölrohmaterialien enthalten Naturölkomponenten, beispielsweise Pflanzenöle und Tieröle, höheren Alkohol oder eine höhere Fettsäure, die gereinigt und von Ölen, Fetten und Wachsen getrennt ist, Kohlenwasserstofföle, die durch Reinigung mittels einer fraktionellen Destillation von Petroleum gewonnen werden, und synthetische Esteröle.

Lanolin ist eines der Tieröle, das durch Reinigung von Ölen und Fetten gewonnen wird, die an Wolle anhaften. Lanolin ist ein benutzbares Ölrohmaterial mit einem großen Wasserhaltevermögen, das nicht anderen Ölen und Fetten eigen ist, die als Ölrohmaterialien eingesetzt werden. Lanolin hat sich schon lange als brauchbares Ölrohmaterial erwiesen. Lanolin ist weit verbreitet für alle Arten von Kosmetikartikeln verwendet worden, beispielsweise zur Hautpflege, Haarpflege und zum Schminken.

Als Esteröle sind diglyzerolverzweigte Fettsäureester, beispielsweise Diglyzerol-Diisostearat und Digyzerol-Triisostearat, und apfelsäureverzweigte Fettsäureester, beispielsweise Diisostearol-Malat, bekannt. Diese Ester werden unter Verwendung von Fettsäuren hergestellt, die von Pflanzenölen abgeleitet sind, und sind als Ölrohmaterialien mit wasserhaltenden Eigenschaften bekannt, beispielsweise Lanolin. Ferner werden diese Ester für verschiedene Arten von Kosmetikartikeln, insbesondere für Lippenkosmetikartikel wie Lippenstifte, verwendet. Nebenbei bemerkt sind diese Ester, die unter Verwendung von Fettsäuren hergestellt werden, die von Pflanzenöl abgeleitet und als Ölrohmaterial für Kosmetikartikel verwendet werden, als Dekaglyzerol-Pentaisostearat bekannt.

In den japanischen Offenlegungsschriften 11/246354 und 2002/255738 sind beispielsweise weitere, üblicherweise verwendete Ölrohmaterialien mit wasserhaltenden Eigenschaften offenbart. Im Absatz 0021 der japanischen Offenlegungsschrift 11/246354 und im Absatz 0010 der japanischen Offenlegungsschrift 2002/255738 sind als Ölrohmaterialien mit wasserhaltenden Eigenschaften Lanolinderivate, Substanzen, die durch Modifizierung von Lanolin mit Polyoxyalkylen, Esteröle auf der Basis von Aminosäure, Polyalkohol-Fettsäureester, Polyglyzerol-Fettsäureester und Hydroxyfettsäureester offenbart.

JP7-308560 beschreibt ein Veresterungsprodukt, das durch Versetzung von Polyglycerol und einer Fettsäure gewonnen wird und dessen Zusammensetzungen.

Wegen des heute in Europa und den Vereinigten Staaten von Amerika zunehmenden Tierschutzes sollte die Verwendung des von Tierölen abstammenden Lanolins durch die Benutzer vermieden werden. Die Verwendung von Ölrohmaterialien, die von Rindertalg abgeleitet sind, das eins der Tieröle außer Lanolin ist, wird mehr von den Verbrauchern auf Grund der legalen Verwendungsbeschränkungen und des heutigen Problems mit der Rinderwahnsinnskrankheit vermieden. Unter diesen Umständen, nämlich unter Vermeidung von Rindertalg, hat auch die Kosmetikindustrie die Verwendung des vom Schaf abgeleiteten Lanolins, das manchmal an der Schafwahnsinnskrankheit (Wundscharren) ähnlich wie der Rinderwahnsinn leidet, vermieden.

Andererseits zeigen zwar die diglyzerolverzweigten Fettsäureester, die unter Verwendung von Pflanzen abgeleiteten Fettsäuren hergestellt werden, Wasserhalteeigenschaften, die aber im Vergleich zu Lanolin unzureichend sind. Auf Grund einer Prüfung der wasserhaltenden Eigenschaft des üblichen Dekaglyzerol-Pentaisostearats, das unter Verwendung einer von Pflanzen abgeleiteten Fettsäure hergestellt wird, liegt die Größe der wasserhaltenden Eigenschaft unterhalb der des Lanolins. Ferner sondert das Dekaglyzerol-Pentaisostearat einen (üblen) Geruch ab und zeigt eine ungenügende Feuchtigkeitsrückhalteeigenschaft, wenn es für Kosmetikartikel verwendet wird.

Weitere Ölrohmaterialien, die in den japanischen Offenlegungsschriften 11/246354 und 2002/255738 konkret offenbart sind, zeigen im Vergleich zu Lanolin nur unzureichende Wasserhalteeigenschaften.

Wegen der vorstehend erwähnten Umstände besteht eine Aufgabe der vorliegenden Erfindung darin, ein Veresterungsprodukt zu schaffen, das ein Ölrohmaterial ist, das unter Verwendung von einer von Pflanzen abgeleiteten Fettsäure hergestellt werden kann, das von den Verbrauchern bevorzugt wird und das eine Wasserhalteeigenschaft und auch eine feuchtigkeitsrückhaltende Eigenschaft zeigt, wenn es als Rohmaterial eines Kosmetikartikels verwendet wird.

Eine weitere Aufgabe der vorliegenden Erfindung besteht darin, einen Kosmetikartikel zu schaffen, der ein Veresterungsprodukt mit einer Wasserhalteeigenschaft und einer Feuchtigkeitsrückhalteeigenschaft umfasst.

Die Erfinder der vorliegenden Erfindung studierten sorgfältig, um die vorerwähnten Probleme zu lösen, und sind als Ergebnis zur vorliegenden Erfindung gekommen; sie haben festgestellt, dass ein Veresterungsprodukt der Fettsäure, das durch Verestern eines besonderen Polyglyzerols und einer besonders verzweigten Fettsäure gewonnen wird und einen bestimmten Veresterungsgrad aufweist, eine Wasserhalteeigenschaft aufweist und ein Veresterungsprodukt darstellt, das für Wasserhalteanwendungen geeignet ist.

Das heißt, dass die vorliegende Erfindung ein Veresterungsprodukt schafft, das durch Verestern von Polyglyzerol und einer Fettsäure gewonnen wird, wobei das Polyglyzerol eine Durchschnittspolymerisierungsgrad von 6-15 aufweist, der auf der Basis des Hydroxylwerts des Polyglyzerols berechnet ist, wobei ferner die Fettsäure mindestens eine verzweigte Fettsäure ist, die aus verzweigten Fettsäuren ausgewählt ist, die 8-22 Kohlenstoffatome aufweisen, und wobei das Veresterungsprodukt einen Veresterungsgrad von 60 % bis 90% und eine Säurezahl von 3,0 oder weniger aufweist. Der Begriff "Vereste-rungsprodukt" umfasst hier sowohl ein Veresterungsprodukt, das eine freie Fettsäure aufweist, als auch ein Veresterungsprodukt, das keine freie Fettsäure aufweist.

Der Durchschnittspolymerisierungsgrad n, der auf der Basis eines Hydroxylwerts berechnet ist, stellt einen Wert dar, der durch eine Endanalyse und durch die folgenden Formeln 1 und 2 errechnet wird:

Formel 1: Molekulargewicht = 74n+18,
Formel 2: Hydroxylwert = 56110(n+2)/Molekulargewicht.

Der Hydroxylwert ist ein Wert, der als Indikator für die Größe der Anzahl der Hydroxylgruppen dient, die in einem Veresterungsprodukt enthalten sind. Es ist die Milligrammzahl von Kaliumhydroxid, das für das Azetylieren von freien Hydroxylgruppen benötigt wird, die in 1 g des Veresterungsprodukts enthalten sind. Die Milligrammzahl des Kaliumhydroxids wird nach den "Standardverfahren für die Analyse von Fetten, Ölen und entsprechenden Materialien (I), vorgeschrieben durch die japanische Ölchemikergesellschaft (1996)" errechnet, das von der japanischen Ölchemikergesellschaft herausgegeben wurde.

Der Veresterungsgrad ist ein Wert, der aus der Formel

$$\text{Veresterungsgrad (\%)} = \{M/(n+2)\}100$$

berechnet wird, wobei

nder Durchschnittspolymerisierungsgrad von Polyglyzerol ist, der auf der Basis eines Hydroxylwerts berechnet wird, n+2die Hydroxylgruppenzahl des Polyglyzerols ist und

Mdie Molzahl der verzweigten Fettsäure ist.

Das Veresterungsprodukt ist ein solches, das eine Säurezahl von 3,0 oder weniger aufweist. Die Säurezahl ist ein Wert, der als Indikator für die Größe der freien Fettsäure ist, die in einem Veresterungsprodukt enthalten ist. Er ist die Milligrammanzahl von Kaliumhydroxid, das zur Neutralisierung der freien Fettsäure benötigt wird, die in 1 g des Veresterungsprodukts enthalten ist, wobei diese Kaliumhydroxidmenge gemäß den "Standardverfahren zur Analyse von Fetten, Ölen und entsprechenden Materialien, vorgeschrieben von der Japanischen Ölchemikergesellschaft (1996)", herausgegeben von der Japanischen Ölchemikergesellschaft, berechnet wird.

Die vorliegende Erfindung ist auf einen Kosmetikartikel gerichtet, das das Veresterungsprodukt enthält.

Das Veresterungsprodukt besitzt eine Wasserhalteeigenschaft und verursacht bei Verwendung als Rohmaterial für einen Kosmetikartikel, dass dieser eine gute Feuchtigkeitsrückhalteeigenschaft aufweist, weil er derart ausgebildet ist, dass ein besonderes Polyglyzerol und eine besonders verzweigte Fettsäure verestert werden und ein Veresterungsgrad von 60 % bis 90% erreicht wird. Wenn das Veresterungsprodukt eine Säurezahl von 3,0 oder weniger aufweist, ist es hinsichtlich des Geruchs überlegen.

Ein Kosmetikartikel, der wie oben beschrieben ausgebildet ist, wird unter Einschluss des Veresterungsprodukts gemäß der Erfindung hergestellt und weist daher eine ausgezeichnete Wasserhalteeigenschaft und Feuchtigkeitsrückhalteeigenschaft auf.

Die vorliegende Erfindung wird im Folgenden anhand von Ausführungsbeispielen näher erläutert. Das Veresterungsprodukt einer verzweigten Fettsäure gemäß der vorliegenden Erfindung wird durch Veresterung von Hydroxylgruppen erzielt, die Polyglyzerol in einer oder mehreren Arten der besonders verzweigten Fettsäure aufweisen.

Als Polyglyzerol wird ein auf der Basis einer Hydroxylzahl berechnetes Polyglyzerol ausgewählt, das einen Durchschnittspolymerisierungsgrad von 6-15, vorzugsweise von 8-12, aufweist.

Als besonders verzweigte Fettsäure werden ein oder mehrere Arten von Fettsäuren ausgewählt, die 8-22, vorzugsweise 14-20, Kohlenstoffatome aufweisen. Die Verwendung einer gesättigten, besonders verzweigten Fettsäure wird bevorzugt. Beispiele für die gesättigten, besonders verzweigten Fettsäuren mit 8-22 Kohlenstoffatomen umfassen 2-Ethylhexansäure, 3,5,5-Trimethylhexansäure, 4-Propylpentansäure, 4-Ethylpentansäure, 2-Methyldekansäure, 3-Methyldekansäure, 4-Methyldkansäure, 5-Methyldekansäure, 6-Methyldekansäure, 7-Methyldekansäure, 9- Methyldekansäure, 6-Ethylennonansäure, 5-Propyloktansäure, 3-Methylundekansäure, 6-Propylnonansäure, 2-Methyldodekansäure, 3-Methyldodekansäure, 4-Methyldodekansäure, 5-Methyldodekansäure, 11-Methyldodekansäure. 7-Propyldekansäure, t2-Methyltridekansäure, 12-Methyltridekansäure, 2-Methyltetradekansäure, 4-Methyltetradekansäure, 13-Methyltetradekansäure, 14-Methylpentadekansäure, 2-Ethyltetradekansäure, 15-Methylhexadekansäure, 2-Propyldekansäure, 2-Ethylhexadekansäure, 14-Ethylhexadekansäure, 14-Methylheptadekansäure, 15-Methylheptadekansäure, 16-Methylheptadekansäure, 2-Butyltetradekansäure, 2-Methyloktadekansäure, 3- Methyloktadekansäure, 4-Methyloktadekansäure, 5-Methyloktadekansäure, 6-Methyloktadekansäure, 7-Methyloktadekansäure, 8-Methyloktadekansäure, 9-Methyloktadekansäure,10-Methyloktadekansäure, 11-Methyloktadekansäure, 14-Methyloktadekansäure,15-Methyloktadekansäure, 16-Methyloktadekansäure, 17-Methyloktadekansäure,15-Ethylpentadekansäure, 3-Methylnonadekansäure, 2-Ethyloktadekansäure, 2-Methyleikosansäure, 2-Propyloktadekansäure und 2-Butyloktadekansäure. Unter den gesättigten, verzweigten Fettsäuren wird 16-Methylheptadekansäure (Isostearinsäure) bevorzugt, die 18 Kohlenstoffatome aufweist.

Das Veresterungsprodukt weist einen Veresterungsgrad von 60 % bis 90% auf, bei dem es eine Wasserhalteeigenschaft aufweist, die größer als die des Lanolins ist. Wenn der Veresterungsgrad geringer als 60 % ist, ist die Lipophilität gering, und die Wasserabstoßung ist ungenügend. In diesem Fall wird die Wirkung der Unterbindung der Feuchtigkeitsabgabe von der Haut vermindert, und wenn das Veresterungsprodukt in einem Kosmetikartikel verwendet wird, ist die Feuchtigkeitsrückhalteeigenschaft des Kosmetikartikels gering. Damit das Veresterungsprodukt in einem Kosmetikartikel verwendet werden kann, das die Feuchtigkeitsrückhalteeigenschaft zur Verbesserung des Hautschutzes verbessert, ist es aus diesem Grund erwünscht, dass der Veresterungsgrad 60 % oder mehr beträgt.

Der Veresterungsgrad ist von 90 % oder weniger, vorzugsweise 80 % oder weniger, betragen. Wenn der Veresterungsgrad mehr als 90 % beträgt, dann geht die Wasserhalteeigenschaft unter der des Lanolins zurück, und wenn er mehr als 80 % beträgt, dann ist die Wasserhalteeigenschaft größer als die des Lanolins. Die Wasserhalteeigenschaft hat dann die Neigung, mit ansteigendem Veresterungsgrad abzunehmen.

Die Säurezahl eines Veresterungsprodukts- beträgt nicht mehr als 3,0, weil es in diesem Fall möglich ist, zu verhindern, dass das Veresterungsprodukt und ein durch Verwendung des Veresterungsprodukts erzeugter Kosmetikartikel einen Geruch abgibt. Wenn ein Veresterungsprodukt eine Säurezahl von mehr als 3,0 aufweist, steigt der Gehalt an ungeänderten Fettsäuren, und deshalb treten hinsichtlich des Geruchs schlechte Wirkungen auf. Ferner besteht die Gefahr, dass durch die ungeänderte Fettsäure eine Hautreizung auftritt. Deshalb ist ein derartiges Veresterungsprodukt nicht als Ölrohmaterial für Kosmetikartikel geeignet.

Das Veresterungsprodukt der verzweigten Fettsäure gemäß der vorliegenden Erfindung wird durch Verwendung des folgenden Verfahrens erzeugt, bei dem das Polyglyzerol mit einem in einem bestimmten Bereich liegenden Durchschnittspolymerisierungsgrad und die verzweigte Fettsäure mit Kohlenstoffatomen, deren Anzahl in einem bestimmten Bereich liegt, miteinander verestert werden. Zuerst wird die verzweigte Fettsäure dem Polyglyzerol zugefügt. In demjenigen Fall, in dem zwei oder mehrere Arten der verzweigten Fettsäuren zugefügt werden, ist es nur nötig, bestimmte Mengen der verzweigten Fettsäuren unabhängig davon zuzufügen, ob die verzweigten Fettsäuren gemischt und dann dem Polyglyzerol zugefügt werden oder die verzweigten Fettsäuren einzeln nacheinander zugefügt werden. Darauf wird ein alkalischer Katalysator wie Seifenstein dem Polyglyzerol zugefügt, dem die verzweigte Fettsäure zugesetzt worden war, und dann wird eine Veresterungsreaktion unter Normaldruck oder einem verminderten Druck gemäß einem bekannten Verfahren ausgeführt. Für die Veresterungsreaktion ist erwünscht, dass sie stetig ausgeführt wird, bis das sich ergebende Veresterungsprodukt einen Veresterungsgrad von 60 % bis 90%, aufweist und eine Säurezahl von 3,0 oder weniger erreicht ist.

Ein Veresterungsprodukt der verzweigten Fettsäure enthaltender Kosmetikartikel gemäß der vorliegenden Erfindung kann mittels eines üblichen Verfahrens zur Präparierung eines Kosmetikartikels, das das Veresterungsprodukt in der Ausführung verwendet, präpariert werden.

Die vorliegende Erfindung wird nun anhand von Ausführungsbeispielen näher erläutert, auf die die Erfindung jedoch nicht beschränkt ist.

Die Veresterungsprodukte der Beispiele 1-3 wurden folgendermaßen präpariert. Die Veresterungsprodukte von Vergleichsbeispielen 1-4 wurden ebenfalls präpariert, die als Bezugsbeispiele für die Beispiele 1-3 dienen. Es sei darauf hingewiesen, dass die Fettsäuren, die für die Präparierungen der Veresterungsprodukte der Beispiele 1-3 und der Vergleichsbeispiele 1-4 verwendet wurden, alle pflanzenabgeleitete Fettsäuren waren.

Beispiel 1:

In einen Reaktor wurden 100 g Polyglyzerol, das einen Durchschnittspolymerisierungsgrad von 10 aufwies, der auf der Basis eines Hydroxylwerts berechnet wurde, und 337 g 16-Methylheptadekansäure (gängige Bezeichnung: Isostearinsäure) eingegeben. Nach der Zufügung von 0,2 g Seifenstein wurde eine Veresterungsreaktion vier Stunden lang bei einer Temperatur von 250° C und unter einer Stickstoffströmung ausgeführt. Auf diese Weise wurden 395 g des Veresterungsprodukts der verzweigten Fettsäure des Beispiels 1 erzeugt. Das Veresterungsprodukt der verzweigten Fettsäure wies einen Veresterungsgrad von 75,0 % und eine Säurezahl von 1,0 auf.

Beispiel 2:

In einen Reaktor wurden 100 g Polyglyzerol, das einen Durchschnittspolymerisierungsgrad von 6 aufwies, der auf der Basis eines Hydroxylwerts berechnet wurde, und 150 g 2-Ethylhexansäure (gängige Bezeichnung: Oktylsäure) eingegeben. Dann wurde eine Reaktion unter denselben Bedingungen wie im Beispiel 1 durchgeführt. Auf diese Weise wurden 220 g des Veresterungsprodukts der verzweigten Fettsäure des Beispiels 2 erzeugt. Das Veresterungsprodukt der verzweigten Fettsäure wies einen Veresterungsgrad von 60,0 % und eine Säurezahl von 0,5 auf.

Beispiel 3:

In einen Reaktor wurden 100 g Polyglyzerol, das einen Durchschnittspolymerisierungsgrad von 12 aufwies, der auf der Basis eines Hydroxylwerts berechnet wurde, und 351 g Isostearinsäure eingegeben. Dann wurde eine Reaktion unter denselben Bedingungen wie im Beispiel 1 durchgeführt. Auf diese Weise wurden 407 g des Veresterungsprodukts der verzweigten Fettsäure des Beispiels 3 erzeugt. Das Veresterungsprodukt der verzweigten Fettsäure wies einen Veresterungsgrad von 80,0 % und eine Säurezahl von 3,0 auf.

Vergleichsbeispiel 1:

In einen Reaktor wurden 100 g Polyglyzerol, das einen Durchschnittspolymerisierungsgrad von 10 aufwies, der auf der Basis eines Hydroxylwerts berechnet wurde, und 225 g Isostearinsäure eingegeben. Dann wurde eine Reaktion unter denselben Bedingungen wie im Beispiel 1 durchgeführt. Auf diese Weise wurden 295 g des Veresterungsprodukts der verzweigten Fettsäure des Vergleichsbeispiels 1 erzeugt. Das Veresterungsprodukt der verzweigten Fettsäure wies einen Veresterungsgrad von 50,0 % und eine Säurezahl von 5,0 auf.

Vergleichsbeispiel 2:

In einen Reaktor wurden 100 g Polyglyzerol, das einen Durchschnittspolymerisierungsgrad von 4 aufwies, der auf der Basis eines Hydroxylwerts berechnet wurde, und 380 g Isostearinsäure eingegeben. Dann wurde eine Reaktion unter denselben Bedingungen wie im Beispiel 1 durchgeführt. Auf diese Weise wurden 433 g des Veresterungsprodukts der verzweigten Fettsäure des Vergleichsbeispiels 2 erzeugt. Das Veresterungsprodukt der verzweigten Fettsäure wies einen Veresterungsgrad von 70,0 % und eine Säurezahl von 1,0 auf.

Vergleichsbeispiel 3:

In einen Reaktor wurden 100 g Polyglyzerol, das einen Durchschnittspolymerisierungsgrad von 10 aufwies, der auf der Basis eines Hydroxylwerts berechnet wurde, und 335 g Oleinsäure eingegeben. Dann wurde eine Reaktion unter denselben Bedingungen wie im Beispiel 1 durchgeführt. Auf diese Weise wurden 393 g des Veresterungsprodukts der verzweigten Fettsäure des Vergleichsbeispiels 3 erzeugt. Das Veresterungsprodukt der verzweigten Fettsäure wies einen Veresterungsgrad von 75,0 % und eine Säurezahl von 1,0 auf.

Vergleichsbeispiel 4:

In einen Reaktor wurden 100 g Polyglyzerol, das einen Durchschnittspolymerisierungsgrad von 10 aufwies, der auf der Basis eines Hydroxylwerts berechnet wurde, und 337 g Stearinsäure eingegeben. Dann wurde eine Reaktion unter denselben Bedingungen wie im Beispiel 1 durchgeführt. Auf diese Weise wurden 395 g des Veresterungsprodukts der verzweigten Fettsäure des Vergleichsbeispiels 4 erzeugt. Das Veresterungsprodukt der verzweigten Fettsäure wies einen Veresterungsgrad von 75,0 % und eine Säurezahl von 1,0 auf.

Die Wasserhalteeigenschaft und der Geruch jedes der Veresterungsprodukte der Beispiele und der Vergleichsbeispiele wurden gemäß einer Prüfung des Prozentsatzes des gehaltenen Wassers und einer Prüfung der Geruchsstärke geprüft, wobei diese beiden Prüfungen im Folgenden beschrieben sind. Beide Prüfungen wurden auch für Lanolin und Diglyzerol-Triisostearat durchgeführt.

Prüfung des Prozentsatzes des gehaltenen Wassers:

Eine Mischung aus 1 g Veresterungsprodukt eines Beispiels, Veresterungsprodukt eines Vergleichsbeispiels, Lanolin oder Digyzerol-Triisostearat und 9 g Vaselin wurde als Probe verwendet. Unter Umrühren der Probe wurde ihr Wasser tropfenweise langsam zugeführt. Auf diese Weise wurde die Probe veranlasst, das Wasser zu halten, bis das Wasser zu verschlicken begann. Der Prozentsatz des gehaltenen Wassers wurde dabei wie folgt bestimmt:

Prozentsatz des gehaltenen Wassers (%) = Gewicht des gehaltenen Wassers (g) durch Gewicht der Probe (g) x 100.

Prüfung der Geruchsstärke:

Auf dem Handrücken wurde 1 g Veresterungsprodukt eines Beispiels, Veresterungsprodukt eines Vergleichsbeispiels, Lanolin oder Diglyzerol-Triisostearat gesprüht, und dann wurde die Geruchsstärke gemäß den unten angegebenen Kriterien sensorisch ausgewertet. Die Anzahl der Beobachter, die die sensorische Auswertung vornahmen, war 20, und zwar Männer und Frauen.

Die Ergebnisse der Prüfung des Prozentsatzes des gehaltenen Wassers und die Ergebnisse der Prüfung der Geruchsstärke sind zusammen mit dem Durchschnittspolymerisierungsgrad des bei der Veresterung verwendeten Polyglyzerols, den Fettsäuren, den Graden und den Säurezahlen der Veresterung des Veresterungsprodukts in der folgenden Tabelle 1 gezeigt.

Tabelle 1

| Probe | Durchschnitts-Polymerisierungsgrad von Polyglyzerol | Fettsäure | Veresterungsgrad (%) | Säurezahl | Wasserhalteprozentsatz (%) | Geruch |
|---|---|---|---|---|---|---|
| Beispiel 1 | 10 | Isostearinsäure | 75,0 | 1,0 | 560 | ○ |
| Beispiel 2 | 6 | Oktylsäure | 60,0 | 0,5 | 510 | ○ |
| Beispiel 3 | 12 | Isostearinsäure | 80,0 | 3,0 | 580 | ○ |
| Vergleichsbeispiel 1 | 10 | Isostearinsäure | 50,0 | 5,0 | 330 | × |
| Vergleichsbeispiel 2 | 4 | Isostearinsäure | 70,0 | 1,0 | 200 | ○ |
| Vergleichsbeispiel 3 | 10 | Oleinsäure | 75,0 | 1,0 | 300 | Δ |
| Vergleichsbeispiel 4 | 10 | Stearinsäure | 75,0 | 1,0 | 180 | ○ |
| Lanolin | | | | | 360 | × |
| Diglyzerol-Triisostearat | | | 75,0 | 1,0 | 290 | ○ |
| Kriterien: O : fast kein Geruch ist wahrnehmbar, Δ : ein leichter Geruch ist wahrnehmbar, X : ein starker Geruch ist wahrnehmbar. | | | | | | |

Aus der Tabelle 1 kann festgestellt werden, dass das Veresterungsprodukt der Beispiele jeweils einen Veresterungsgrad von 60 % und mehr aufweist und jeweils durch Veresterung eines Polyglyzerols mit einer verzweigten Fettsäure erzielt wird, wobei das Polyglyzerol einen Durchschnittspolymerisierungsgrad von 6 bis15 und die Fettsäure 8 bis 22 Kohlenstoffatome aufweist. Das Veresterungsprodukt weist jeweils einen Wasserhalteprozentsatz auf, der größer als der des Veresterungsprodukts der Vergleichsbeispiele ist und der um 200 % höher als der von Lanolin ist.

Es kann aus der Tabelle 1 ebenfalls festgestellt werden, dass das Veresterungsprodukt des Vergleichsbeispiels 1, das eine Säurezahl von 5,0 aufweist, einen stark wahrnehmbaren Geruch abgibt, dass das Veresterungsprodukt des Vergleichsbeispiels 3, das eine Säurezahl von 3,0 aufweist, ebenfalls einen stark wahrnehmbaren Geruch abgibt und dass aber die Veresterungsprodukte aller Beispiele, die Säurezahlen von nicht mehr als 3,0 aufweisen, überlegen sind, weil sie fast keinen wahrnehmbaren Geruch abgeben. Ferner kann aus der Tabelle 1 ebenfalls festgestellt werden, dass Lanolin, das einen höheren Wasserhalteprozentsatz als alle anderen Veresterungsprodukte der Vergleichsbeispiele 1-4 aufweist, einen stark wahrnehmbaren Geruch abgibt, doch haben die Veresterungsprodukte der Beispiele 1-3 der vorliegenden Erfindung die einzigartigen Eigenschaften, dass ihr Wasserhalteprozentsatz größer als der von Lanolin ist und dass sie fast keinen wahrnehmbaren Geruch abgeben.

Getrennt von den Veresterungsprodukten der Beispiele 1 bis 3 wurden Veresterungsprodukte von Beispielen 4 bis 7 präpariert, wobei pflanzenabgeleitete Fettsäuren folgendermaßen verwendet wurden.

Beispiel 4:

In einen Reaktor wurden 100 g Polyglyzerol, das einen Durchschnittspolymerisierungsgrad von 10 aufwies, der auf der Basis eines Hydroxylwerts berechnet wurde, und 337 g Isostearinsäure eingegeben. Dann wurde eine Reaktion unter denselben Bedingungen wie im Beispiel 1 durchgeführt. Auf diese Weise wurde ein Veresterungsprodukt der verzweigten Fettsäure des Vergleichsbeispiels 4 erzeugt.

Beispiel 5:

Ein Veresterungsprodukt der verzweigten Fettsäure des Beispiels 5 wurde in derselben Weise wie im Beispiel 4 erzeugt, mit der Ausnahme, dass die Menge der Isostearinsäure auf 360 g geändert wurde.

Beispiel 6:

Ein Veresterungsprodukt der verzweigten Fettsäure des Beispiels 6 wurde in derselben Weise wie im Beispiel 4 erzeugt, mit der Ausnahme, dass die Menge der Isostearinsäure auf 405 g geändert wurde.

Beispiel 7:

Ein Veresterungsprodukt der verzweigten Fettsäure des Beispiels 7 wurde in derselben Weise wie im Beispiel 4 erzeugt, mit der Ausnahme, dass die Menge der Isostearinsäure auf 450 g geändert wurde.

Die Wasserhalteprozentsätze der sich ergebenden Veresterungsprodukte der Beispiele 4-7 wurden in derselben Weise geprüft, wie sie oben beschrieben wurden. Die Ergebnisse sind in der folgenden Tabelle 2 dargestellt.

Tabelle 2

| Probe | Durchschnittspolymerisierungsgrad von Polyglyzerol | Fettsäure | Veresterungsgrad (%) | Wasserhalteprozentsatz (%) |
|---|---|---|---|---|
| Beispiel 4 | 10 | Isostearinsäure | 75 | 540 |
| Beispiel 5 | 10 | Isostearinsäure | 80 | 500 |
| Beispiel 6 | 10 | Isostearinsäure | 90 | 360 |
| Beispiel 7 | 10 | Isostearinsäure | 100 | 230 |

In der Tabelle 2 sind die Wasserhalteprozentsätze der Veresterungsprodukte mit Ausnahme des Beispiels 7 höher als der Wasserhalteprozentsatz (360 %) von Lanolin, der in der Tabelle 1 angegeben ist. Mit anderen Worten kann festgestellt werden, dass die Veresterungsprodukte der Beispiele 4-7 gemäß der vorliegenden Erfindung und die Veresterungsprodukt gemäß der vorliegenden Erfindung, die Veresterungsgrade von weniger als 90 % haben, Wasserhalteeigenschaften aufweisen, die gleich oder größer als die von Lanolin sind. Ferner kann festgestellt werden, dass die Veresterungsprodukte gemäß der vorliegenden Erfindung, wenn der Veresterungsgrad größer als 80 % ist, dazu neigen, verminderte Wasserhaltegrade zu zeigen.

Präparierung von Kosmetikartikeln:

Verschiedene Arten von Kosmetikartikeln wurden durch Vermischung mit den Veresterungsprodukten der Beispiele 1-3 oder der Vergleichsbeispiele präpariert. Die Präparierungen der Kosmetikartikel wurde unter Verwendung von Mischungsverhältnissen und Verfahren ausgeführt, die in den folgenden Rezepturbeispielen 1-9 und Vergleichsrezepturbeispielen 1-9 angegeben sind.

Rezepturbeispiel 1 (Weichmachende Creme):

| Phase A | (Gew.-%) |
|---|---|
| Veresterungsprodukt der verzweigten Fettsäure des Beispiels 1 | 5,00 |
| Squalen | 7,50 |
| Dekaglyzerol-Monomyristat | 2,00 |
| Stearinsäure | 3,50 |
| Glyzerol-Monostearat | 2,00 |
| Glyzerol-Tri-2-Ethylhexansäure | 5,00 |
| | |
| Phase B | |
| Glyzerol | 7,00 |
| 10 Gew.-% Wässrige Lösung von Kaliumhydroxid | 1,00 |
| Gereinigtes Wasser | 67,00 |

Die Phase A wurde bei einer Temperatur von 80° C aufgelöst. Dann wurde die auf eine Temperatur von 80° C erwärmte Phase B zur Emulgierung langsam dazugefügt. Der Emulgierung folgte eine Abkühlung auf eine Temperatur von 35° C, um die weichmachende Creme zu erhalten.

Rezepturbeispiel 2 (Milchige Lotion):

| Phase A | (Gew.-%) |
|---|---|
| Veresterungsprodukt der verzweigten Fettsäure des Beispiels 1 | 2,50 |
| Dekaglyzerol-Monooleat | 1,00 |
| | |
| Phase B | |
| 1 Gew.-% Wässrige Lösung von Karboxyvinyl-Polymer | 5,00 |
| 10 Gew.-% Wässrige Lösung von Kaliumhydroxid | 1,00 |
| 1,3-Butylenglykol | 5.00 |
| Glyzerol | 2,00 |
| Gereinigtes Wasser | 83,50 |

Die Phase A wurde bei einer Temperatur von 80° C aufgelöst. Dann wurde die auf eine Temperatur von 80° C erwärmte Phase B zur Emulgierung langsam dazugefügt. Der Emulgierung folgte eine Abkühlung auf eine Temperatur von 35° C, um die milchige Lotion zu erhalten.

Rezepturbeispiel 3 (Lippenstift):

| Phase A | (Gew.-%) |
|---|---|
| Veresterungsprodukt der verzweigten Fettsäure des Beispiels 2 | 20,00 |
| Ceresin | 23,50 |
| Castoröl | 27,00 |
| Flüssiges Paraffin | 15,00 |
| Karnaubawachs | 7,00 |
| Kandelillawachs | 5,00 |
| | |
| Phase B | |
| Titanoxid | 2,00 |
| Rotpigment | 0,50 |

Die Phase A wurde zum homogenen Lösen erwärmt. Die sich ergebende Lösung wurde gekühlt und mit einer Rolle gleichmäßig durchgeknetet. Danach wurde die Phase B zugegeben, wonach eine Entschäumung folgte. Das Ergebnis wurde in eine Form gegossen und schnell gekühlt, so dass sich ein Lippenstift ergab.

Rezepturbeispiel 4 (Shampoo):

| | (Gew.-%) |
|---|---|
| Veresterungsprodukt der verzweigten Fettsäure des Beispiels 2 | 2,0 |
| Glyzerol | 5,00 |
| POE(2)-Laurylether-Natriumsulfat (27 Gew.-% wässrige Lösung) | 20,00 |
| POE(2)-Laurylether-Schwefelsäure-Triethanolamin-Salz | |
| (32 Gew.-% wässrige Lösung) | 35,00 |
| Polyoxypropylen(36)-Methyldiethylammoniumchlorid | 2,00 |
| Palmkernfettsäuren-Diethanolamid (1) | 1,00 |
| Natriumchlorid | 0,50 |
| Zitronensäuren-Monohydrat | 0,20 |
| Gereinigtes Wasser | 34,30 |

Alle Bestandteile wurden zum homogenen Lösen auf eine Temperatur von 80° C erwärmt. Die sich ergebende Lösung wurde auf eine Temperatur von 35° C heruntergekühlt, so dass sich das Shampoo ergab.

Rezepturbeispiel 5 (Haarformungsmittel):

| Phase A | (Gew.-%) |
|---|---|
| Veresterungsprodukt der verzweigten Fettsäure des Beispiels 3 | 3,00 |
| Stearyltrimethylammoniumchlorid (63 Gew.-% wässrige Lösung) | 0,70 |
| Behenyltrimethylammoniumchlorid (80 Gew.-% wässrige Lösung) | 0,60 |
| Stearylalkohol | 2,50 |
| Lipophilglyzerolmonostearat | 0,50 |
| | |
| Phase B | |
| Hydroxyethylzellulose | 0,50 |
| Gereinigtes Wasser | 92,20 |

Die Phase A wurde bei einer Temperatur von 80° C aufgelöst. Dann wurde die auf eine Temperatur von 80° C erwärmte Phase B zur Emulgierung langsam dazugefügt. Der Emulgierung folgte eine Abkühlung auf eine Temperatur von 35° C, um das Haarformungsmittel zu erhalten.

Rezepturbeispiel 6 (Salbengrundlage):

| Phase A | (Gew.-%) |
|---|---|
| Veresterungsprodukt der verzweigten Fettsäure des Beispiels 3 | 10,00 |
| Flüssiges Paraffin | 13,00 |
| Vaselin | 10,00 |
| Cetylalkohol | 10,00 |
| Dekaglyzerolmonoisostearat | 3,00 |
| | |
| Phase B | |
| Natriumlaurylsulfat | 1,00 |
| Gereinigtes Wasser | 53,00 |

Die Phase A wurde bei einer Temperatur von 80° C aufgelöst. Dann wurde die auf eine Temperatur von 80° C erwärmte Phase B zur Emulgierung langsam dazugefügt. Der Emulgierung folgte eine Abkühlung auf eine Temperatur von 35° C, um die Salbengrundlage zu erhalten.

Rezepturbeispiel 7 (Reinigungscreme):

| Phase A | (Gew.-%) |
|---|---|
| Veresterungsprodukt der verzweigten Fettsäure des Beispiels 1 | 20,00 |
| Squalen | 10,00 |
| Isononanylisononanoat | 10,00 |
| Cetylalkohol | 3,00 |
| Lipophilglyzerolmonostearat | 3,00 |
| | |
| Phase B | |
| Dekaglyzerolmonoisostearat | 3,00 |
| Glyzerol | 5,00 |
| Natrium-N-stearoyl-L-glutamat | 1,00 |
| Gereinigtes Wasser | 45,00 |

Die Phase A und die Phase B wurden bei einer Temperatur von 80° C aufgelöst. Dann wurde die Phase A zur Phase B zur Emulgierung langsam dazugefügt. Der Emulgierung folgte eine Abkühlung auf eine Temperatur von 35° C, um die Reinigungscreme zu erhalten.

Rezepturbeispiel 8 (Haarwachs):

| Phase A | (Gew.-%) |
|---|---|
| Veresterungsprodukt der verzweigten Fettsäure des Beispiels 2 | 10,00 |
| Jojobawachs | 3,00 |
| Vaselin | 3,00 |
| Squalen | 2,00 |
| Cetylalkohol | 1,50 |

(fortgesetzt)

| Phase A | (Gew.-%) |
|---|---|
| Selbstemulgierendes Glyzerolmonostearat | 2,00 |

| Phase B | |
|---|---|
| Diglyzerol | 5,00 |
| Dekaglyzerolmonomyristat | 2,00 |
| 2 Gew-% Wässrige Lösung des Akrylsäure-Alkylmethakrylat-Copolymers | 18,00 |
| 2 Gew.-% wässrige Lösung des Karboxyvinyl-Polymers | 5,00 |
| 10 Gew.-% Wässrige Lösung des Kaliumhydroxids | 1,80 |
| Gereinigtes Wasser | 46,70 |

Die Phase A und die Phase B wurden bei einer Temperatur von 80° C aufgelöst. Dann wurde die Phase A zur Phase B zur Emulgierung langsam dazugefügt. Der Emulgierung folgte eine Abkühlung auf eine Temperatur von 35° C, um das Haarwachs zu erhalten.

Rezepturbeispiel 9 (Auf Öl basierende Grundlage; Stifttyp):

| Phase A | (Gew.-%) |
|---|---|
| Veresterungsprodukt der verzweigten Fettsäure des Beispiels 3 | 15,00 |
| Festes Paraffin | 7,50 |
| Mikrokristallines Wachs | 7,00 |
| Diisostearylmalat | 5,00 |
| Phytosteryloleat | 5,00 |
| Gereinigtes Wasser | 10,00 |

| Phase B | |
|---|---|
| Kaolin | 23,00 |
| Titanoxid | 23,00 |
| Rotes Eisenoxid | 1,00 |
| Gelbes Eisenoxid | 3,00 |
| Schwarzes Eisenoxid | 0,50 |

Die Phase A wurde bei einer Temperatur von 85° C homogen aufgelöst. Dann wurde zu der sich ergebenden Lösung die Phase B, die gemischt und pulverisiert worden war, unter Umrühren dazugefügt, wonach ein Mahlen der Dispersion in einer Kolloidmühle folgte. Nach einer Entgasung wurde das Ergebnis in eine Form bei einer Temperatur von 70° C gegossen und schnell abgekühlt, so dass die auf Öl basierende Grundlage erhalten wurde.

Vergleichsrezepturbeispiel 1 (Weichmachende Creme):

| Phase A | (Gew.-%) |
|---|---|
| Veresterungsprodukt des Vergleichsbeispiels 1 | 5,00 |
| Squalen | 7,50 |
| Dekaglyzerolmonomyristat | 2,00 |
| Stearinsäure | 3,50 |
| Glyzerolmonostearat | 2,00 |
| Glyzerol-Tri-2-Ethylhexansäure | 5,00 |
| Phase B | |
| Glyzerol | 7,00 |

(fortgesetzt)

| Phase B | |
|---|---|
| 10 Gew.-% Wässrige Lösung des Kaliumhydroxids | 1,00 |
| Gereinigtes Wasser | 67,00 |

Die Phase A wurde bei einer Temperatur von 80° C aufgelöst. Dann wurde dieser Lösung die auf eine Temperatur von 80° C erwärmte Phase B zur Emulgierung langsam dazugegeben. Der Emulgierung folgte eine Abkühlung auf eine Temperatur von 35° C, so dass sich die weichmachende Creme ergab.

Vergleichsrezepturbeispiel 2 (Milchige Lotion):

| Phase A | (Gew.-%) |
|---|---|
| Veresterungsprodukt des Vergleichsbeispiels 2 | 2,50 |
| Dekaglyzerol-Monooleat | 1,00 |
| | |
| Phase B | |
| 1 Gew.-% Wässrige Lösung von Karboxyvinyl-Polymer | 5,00 |
| 10 Gew.-% Wässrige Lösung von Kaliumhydroxid | 1,00 |
| 1,3-Butylenglykol | 5.00 |
| Glyzerol | 2,00 |
| Gereinigtes Wasser | 83,50 |

Die Phase A wurde bei einer Temperatur von 80° C aufgelöst. Dann wurde die auf eine Temperatur von 80° C erwärmte Phase B zur Emulgierung langsam dazugefügt. Der Emulgierung folgte eine Abkühlung auf eine Temperatur von 35° C, um die milchige Lotion zu erhalten.

Vergleichsrezepturbeispiel 3 (Lippenstift):

| Phase A | (Gew.-%) |
|---|---|
| Veresterungsprodukt des Vergleichsbeispiels 3 | 20,00 |
| Ceresin | 23,50 |
| Castoröl | 27,00 |
| Flüssiges Paraffin | 15,00 |
| Karnaubawachs | 7,00 |
| Kandelillawachs | 5,00 |
| | |
| Phase B | |
| Titanoxid | 2,00 |
| Rotpigment | 0,50 |

Die Phase A wurde zum homogenen Lösen erwärmt. Die sich ergebende Lösung wurde gekühlt und mit einer Rolle gleichmäßig durchgeknetet. Danach wurde die Phase B zugegeben, wonach eine Entschäumung folgte. Das Ergebnis wurde in eine Form gegossen und schnell gekühlt, so dass sich der Lippenstift ergab.

Vergleichsrezepturbeispiel 4 (Shampoo):

| | (Gew.-%) |
|---|---|
| Veresterungsprodukt des Vergleichsbeispiels 4 | 2,00 |
| Glyzerol | 5,00 |
| POE(2)-Laurylether-Natriumsulfat (27 Gew.-% wässrige Lösung) | 20,00 |
| POE(2)-Laurylether-Schwefelsäure-Triethanolamin-Salz (32 Gew.-% wässrige Lösung) | 35,00 |
| Polyoxypropylen(36)-Methyldiethylammoniumchlorid | 2,00 |
| Palmkernfettsäuren-Diethanolamid (1) | 1,00 |
| Natriumchlorid | 0,50 |
| Zitronensäuren-Monohydrat | 0,20 |
| Gereinigtes Wasser | 34,30 |

Alle Bestandteile wurden zum homogenen Lösen auf eine Temperatur von 80° C erwärmt. Die sich ergebende Lösung wurde auf eine Temperatur von 35° C heruntergekühlt, so dass sich das Shampoo ergab.

Vergleichsrezepturbeispiel 5 (Haarformungsmittel):

| Phase A | (Gew.-%) |
|---|---|
| Veresterungsprodukt des Vergleichsbeispiels 1 | 3,00 |
| Stearyltrimethylammoniumchlorid (63 Gew.-% wässrige Lösung) | 0,70 |
| Behenyltrimethylammoniumchlorid (80 Gew.-% wässrige Lösung) | 0,60 |
| Stearylalkohol | 2,50 |
| Lipophilglyzerolmonostearat | 0,50 |
| | |
| Phase B | |
| Hydroxyethylzellulose | 0,50 |
| Gereinigtes Wasser | 92,20 |

Die Phase A wurde bei einer Temperatur von 80° C aufgelöst. Dann wurde die auf eine Temperatur von 80° C erwärmte Phase B zur Emulgierung langsam dazugefügt. Der Emulgierung folgte eine Abkühlung auf eine Temperatur von 35° C, um das Haarformungsmittel zu erhalten.

Vergleichsrezepturbeispiel 6 (Salbengrundlage):

| Phase A | (Gew.-%) |
|---|---|
| Veresterungsprodukt des Vergleichsbeispiels 2 | 10,00 |
| Flüssiges Paraffin | 13,00 |
| Vaselin | 10,00 |
| Cetylalkohol | 10,00 |
| Dekaglyzerolmonomyristat | 3,00 |
| | |
| Phase B | |
| Natriumlaurylsulfat | 1,00 |
| Gereinigtes Wasser | 53,00 |

Die Phase A wurde bei einer Temperatur von 80° C aufgelöst. Dann wurde die auf eine Temperatur von 80° C erwärmte Phase B zur Emulgierung langsam dazugefügt. Der Emulgierung folgte eine Abkühlung auf eine Temperatur von 35° C, um die Salbengrundlage zu erhalten.

Vergleichsrezepturbeispiel 7 (Reinigungscreme):

| Phase A | (Gew.-%) |
|---|---|
| Veresterungsprodukt des Vergleichsbeispiels 3 | 20,00 |
| Squalen | 10,00 |
| Isononanylisononanoat | 10,00 |
| Cetylalkohol | 3,00 |
| Lipophilglyzerolmonostearat | 3,00 |
| | |
| Phase B | |
| Dekaglyzerolmonolaurat | 3,00 |
| Glyzerol | 5,00 |
| Natrium-N-stearoyl-L-glutamat | 1,00 |
| Gereinigtes Wasser | 45,00 |

Die Phase A und die Phase B wurden bei einer Temperatur von 80° C aufgelöst. Dann wurde die Phase A zur Phase B zur Emulgierung langsam dazugefügt. Der Emulgierung folgte eine Abkühlung auf eine Temperatur von 35° C, um die Reinigungscreme zu erhalten.

Vergleichsrezepturbeispiel 8 (Haarwachs):

| Phase A | (Gew.-%) |
|---|---|
| Veresterungsprodukt des Vegleichsbeispiels 4 | 10,00 |
| Jojobawachs | 3,00 |
| Vaselin | 3,00 |
| Squalen | 2,00 |
| Cetylalkohol | 1,50 |
| Selbstemulgierendes Glyzerolmonostearat | 2,00 |
| | |
| Phase B | |
| Diglyzerol | 5,00 |
| Dekaglyzerolmonomyristat | 2,00 |
| 2 Gew-% Wässrige Lösung des Akrylsäure-Alkylmethakrylat-Copolymers | 18,00 |
| 2 Gew.-% Wässrige Lösung des Karboxyvinyl-Polymers | 5,00 |
| 10 Gew.-% Wässrige Lösung des Kaliumhydroxids | 1,80 |
| Gereinigtes Wasser | 46,70 |

Die Phase A und die Phase B wurden bei einer Temperatur von 80° C aufgelöst. Dann wurde die Phase A zur Phase B zur Emulgierung langsam dazugefügt. Der Emulgierung folgte eine Abkühlung auf eine Temperatur von 35° C, um das Haarwachs zu erhalten.

Vergleichsrezepturbeispiel 9 (Auf Öl basierende Grundlage; Stifttyp):

| Phase A | (Gew.-%) |
|---|---|
| Veresterungsprodukt des Vergleichsbeispiels 1 | 15,00 |
| Festes Paraffin | 7,50 |
| Mikrokristallines Wachs | 7,00 |
| Diisostearylmalat | 5,00 |

(fortgesetzt)

| Phase A | (Gew.-%) |
|---|---|
| Phytosteryloleat | 5,00 |
| Gereinigtes Wasser | 10,00 |
| Phase B | |
| Kaolin | 23,00 |
| Titanoxid | 23,00 |
| Rotes Eisenoxid | 1,00 |
| Gelbes Eisenoxid | 3,00 |
| Schwarzes Eisenoxid | 0,50 |

Die Phase A wurde bei einer Temperatur von 85° C homogen aufgelöst. Dann wurde zu der sich ergebenden Lösung die Phase B, die gemischt und pulverisiert worden war, unter Umrühren dazugefügt, wonach ein Mahlen der Dispersion in einer Kolloidmühle folgte. Nach einer Entgasung wurde das Ergebnis in eine Form bei einer Temperatur von 70° C gegossen und schnell abgekühlt, so dass die auf Öl basierende Grundlage erhalten wurde.

Unter Verwendung von Kosmetikartikeln der Rezepturbeispiele und der Vergleichsrezepturbeispiele wurden Auswertungen der Feuchtigkeitsrückhalteeigenschaft (Feuchtegefühl), des Fettsgefühls, der Klebrigkeit und des Geruchs vorgenommen. Diese Auswertungen wurden durch direkte Vergleiche der weichmachenden Creme des Rezepturbeispiels 1 mit der des Vergleichsrezepturbeispiels 1, der milchigen Lotion des Rezepturbeispiels 2 mit der des Vergleichsrezepturbeispiels 2, des Lippenstifts des Rezepturbeispiels 3 mit dem des Vergleichsrezepturbeispiels 3, des Shampoos des Rezepturbeispiels 4 mit dem des Vergleichsrezepturbeispiels 4, des Haarformungsmittels des Rezepturbeispiels 5 mit dem des Vergleichsrezepturbeispiels 5, der Salbengrundlage des Rezepturbeispiels 6 mit der des Vergleichsrezepturbeispiels 6, der Reinigungscreme des Rezepturbeispiels 7 mit der des Vergleichsrezepturbeispiels 7, des Haarwachses des Rezepturbeispiels 8 mit dem des Vergleichsrezepturbeispiels 8 und der auf Öl basierenden Grundlage des Rezepturbeispiels 9 mit der des Vergleichsrezepturbeispiels 9 durchgeführt.

Bei jedem direkten Vergleich eines Rezepturbeispiels mit einem Vergleichsrezepturbeispiel wurde bestätigt, dass ein Kosmetikartikel eines Vergleichsrezepturbeispiels sensorisch unbefriedigend war, weil das Feuchtegefühl und das klebrige Fettgefühl ungenügend waren, wogegen ein Kosmetikartikel eines Rezepturbeispiels hinsichtlich des Feuchtegefühls ausgezeichnet war und beim Gebrauch ein Glättegefühl ohne ein Fettigkeitsgefühl bot. Die Kosmetikartikel der Vergleichsrezepturbeispiele 1, 3, 5, 7 und 9, die unter Verwendung der geruchsverbreitenden Veresterungsprodukteder Vergleichsbeispiele 1 oder 3 präpariert waren, gaben einen Geruch ab. Im Gegensatz dazu gaben nicht nur die Kosmetikartikel der Rezepturbeispiele 1, 3, 5, 7 und 9, sondern die Kosmetikartikel aller Rezepturbeispiele, die unter Verwendung der Veresterungsprodukte gemäß der vorliegenden Erfindung präpariert waren, fast keinen lästigen Geruch ab, und sie sind Produkte, die in zufriedenstellender Weise als geruchslose Kosmetikartikel verwendet werden können.

Industrielle Verwertbarkeit

Aus dem Veresterungsprodukt von verzweigten Fettsäuren gemäß der vorliegenden Erfindung können Kosmetikartikel hergestellt werden, die hohe Feuchtigkeitsrückhalteeigenschaften aufweisen, wenn die Veresterungsprodukte als Ölrohmaterialien für die Kosmetikartikel verwendet werden. Die Veresterungsprodukte gemäß der vorliegenden Erfindung können als Ölrohmaterialien für verschiedene Kosmetikartikel verwendet werden, beispielsweise als Hautpflegemittel, nämlich Cremes und Lotions, Schminkkosmetikartikel, beispielsweise Lippenstifte und Grundlagen, Haarkosmetikartikel, beispielsweise Shampoo, Formungsmittel und Haarwachs, Reinigungskosmetikartikel und Salben.

**Patentansprüche**

1. Veresterungsprodukt, das durch Veresterung von Polyglyzerol und einer Fettsäure gewonnen wird, **dadurch gekennzeichnet,** **dass** das Polyglyzerol einen Durchschnittspolymerisierungsgrad von 6 bis 15 aufweist, der auf der Basis des Hydroxylwerts des Polyglyzerols berechnet ist, dass die Fettsäure aus verzweigten Fettsäuren ausgewählt ist, die 8 bis 22 Kohlenstoffatome aufweisen, und dass das Veresterungsprodukt einen Veresterungsgrad von 60 % bis 90 % und eine Säurezahl von 3,0 oder weniger aufweist.

2. Kosmetikartikel, dass das Veresterungsprodukt nach Anspruch 1 aufweist.

## Claims

1. An esterification product, which is obtained by esterification of polyglycerol and a fatty acid, **characterized in that** the polyglycerol has a mean degree of polymerization of 6 to 15, which is calculated on the basis of the hydroxyl value of the polyglycerol; that the fatty acid is selected from branched fatty acids that have from 8 to 22 carbon atoms; and that the esterification product has a degree of esterification of from 60% to 90% and an acid value of 3,0 or less.

2. A cosmetic article that has the esterification product of claim 1.

## Revendications

1. Produit d'estérification, qui est obtenu par estérification de polyglycérol et d'un acide gras, **caractérisé en ce que** le polyglycérol présente un degré moyen de polymérisation allant de 6 à 15, qui est calculé sur la base de la valeur hydroxyle du polyglycérol, **en ce que** l'acide gras est sélectionné parmi des acides gras ramifiés qui présentent 8 à 22 atomes de carbone, et **en ce que** le produit d'estérification présente un degré d'estérification allant de 60 % à 90 % et un indice d'acidité de 3,0 ou moins.

2. Article cosmétique, qui présente le produit d'estérifcation selon la revendication 1.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 11246354 A
- JP 2002255738 A
- JP 7308560 A

**In der Beschreibung aufgeführte Nicht-Patentliteratur**

- **FETTEN.** Standardverfahren zur Analyse. Japanischen Ölchemikergesellschaft, 1996